# EUROPEAN PATENT APPLICATION

(11) **EP 2 172 189 A1**
(43) Date of publication of application: **07.04.2010**
(21) Application number: 08165662.1
(22) Date of filing: 01.10.2008
(51) Int. Cl.: A61K 9/00, A61K 47/10, A61K 47/34

(54) **Pharmaceutical Compositions**

(71) Applicant: Novartis AG, 4002 Basel (CH)
(72) Inventor: The designation of the inventor has not yet been filed
(74) Representative: Gruber, Markus

(57) **Abstract**

A liquid pharmaceutical composition comprising a biodegradable polymer, polyethylene glycol having a molecular weight of less than 600 Daltons, a pharmaceutically active agent and less than 0.5 % of an biologically acceptable organic solvent.

## Description

The present invention relates pharmaceutical compositions, more specifically to injectable in situ forming depot formulations comprising a pharmaceutically active agent and to a process for preparing said depot formulations.

In-situ forming depots represent a specific class of polymeric delivery systems with the advantages of a straightforward manufacturing even for sensitive molecules and ease of application since the polymer solidifies after application by phase separation. When based on a biodegradable polymer like the frequently used poly (D,L lactide- co- glycolide)s (PLGA), the depot degrades over the time. Currently, there are two injectable in-situ forming depots on the market: Atridox® and Eligard®. Both products were developed on Atrigel's technology by Dunn et al. (H. B. Ravivarapu, K. L. Moyer, R. L. Dunn, International Journal of Pharmaceutics, 194 (2000) 181-191) comprising PLGA dissolved in 1-methyl-2-pyrrolidinone (NMP), a water miscible solvent and a drug substance powder to suspend in the solution prior to application.

Whereas injectable in-situ forming depots is an attractive, it is at the same time also a challenging application system. Besides chemical compatibility, local tolerability and acute toxicity, an important factor for an injectable in-situ forming depot is its storage stability as a liquid.

The present invention now provides in situ forming depot pharmaceutical formulations with improved characteristics with respect to tolerability and acute toxicity as well as storage stability and which are convenient to use, and which are particularly suitable for ready to use injectable depot formulations.

The present invention provides in one aspect an in situ forming depot pharmaceutical formulation comprising
(1) a hydrophobic or hydrophilic pharmaceutically active agent
(2) a biodegradable polymer,
(3) a biocompatible water miscible solvent which has a solidification point at a temperature between 8° to 20°C, preferably a poly (ethylene) glycol with a molecular weight 450<Mw< 650Da and with chemically inert end groups, preferably alkoxy endgroups, more preferred ethoxy and methoxy endgroups and optionally
(4) an additive.

The biocompatible water miscible solvent is preferably chosen among PEGs with a solidification point between 8° to 20°C, preferably between between 10° to 16°C to obtain a stable solid formulation at low temperature to avoid sedimentation of drug substance particles. In one embodiment the solidification point is <15°C. The PEG used as solvent has inert end groups to obtain improved stability as well as lower viscosity compared to non end-capped PEGs of same molecular weight. In a preferred embodiment, polyethylene glycol mono and di-alkyl ether such as e.g. dimethylether or diethylether PEGs are used. In a another preferred embodiment, the molecular weight of the end-capped PEG is between 500 and 600 Da, in a more preferred embodiment the Mw is 450, 500, 550 or 650 Da. In a particularly preferred embodiment, the PEG is chosen from polyethylene glycol 500 dimethylether (melting temperature ca.13°C). The PEGs of the present invention show low hemolysis and toxicity potential.

The pharmaceutical compositions of the present invention are in a solid state at storage temperatures of 2-8°C (typical refrigerator temperature) and are liquid and free or substantially free from sedimentation at room temperature. The pharmaceutical compositions (without drug substance) of the present invention have a viscosity suitable for an easy injection, e.g. injectable s.c. or i.m., when equilibrated to room temperature. The dynamic viscosity of the polymer solution (pharmaceutical composition without drug substance) is preferably between 300 and 800 mPas. The pharmaceutical compositions of the present invention are particularly useful for ready to use devices because no re-suspending step is required prior to application.

The pharmaceutically active agent may be dissolved or dispersed in liquid polyethylene glycols (PEG) with modified end groups as described above e.g. polyethylene glycol mono (USP 31 NF26) and di-alkyl ether e.g. at room temperature (e.g. 25° C), e.g. depending on its solubility in this solvent with or without a co-solvent. In a preferred embodiment no co-solvent, such as e.g. an organic solvent, is used.

The pharmaceutically active agent (also interchangeably called drug substance herein) can be a hydrophilic or hydrophobic, small organic molecule, peptide or protein. The pharmaceutically active agent can be in form of the free base, free acid or a salt.

Examples of pharmaceutically active agents include but are not limited to peptides, polypeptides, proteins, carbohydrates, oligonucleotides, RNA and DNA. A few examples of peptides are antibodies, growth hormones, e.g. epidermal growth factor (EGF), prolactin, luliberin or luteinizing hormone releasing hormone (LH-RH), glucagon, gastrin, pentagastrin, urogastron, secretin, enkephalins, endorphins, angiotensins, renin, bradykinin, bacitracins, polymyxins, colistins, tyrocidin, gramicidines, insulin, interferons, erythropoietin, calcitonin, heparin, somatostatin analogues, e.g. somatostatin pamoate or di-aspartate, cell stimulating factors and parathyroid hormones. Other examples include bisphosphonates such as e.g. pamidronic acid, alendronic acid, ibandronic acid, risedronic acid, zoledronic acid.

A preferred active agent may be a somatostatin analogue. Somatostatin is a tetradecapeptide having the structure

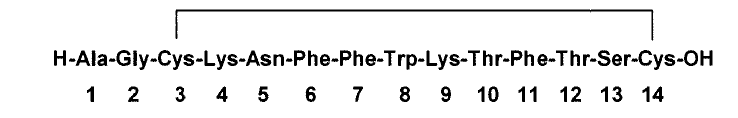

Somatostatin analogues of particular interest include octreotide, e.g. as disclosed in US 4,395,403, lanreotide or pasireotide. Somatostatin analogues of particular interest have also been described e.g. in WO97/01579 and WO02/010192. Said somatostatin analogues comprise the amino acid sequence of formula I

-(D/L)Trp-Lys-X₁ -X₂ - I

wherein X₁ is a radical of formula (a) or (b) 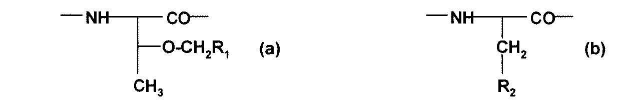 wherein R₁ is optionally substituted phenyl, wherein the substituent may be halogen, methyl, ethyl, methoxy or ethoxy,

R₂ is -Z₁-CH₂-R₁, -CH₂-CO-O-CH₂-R₁,

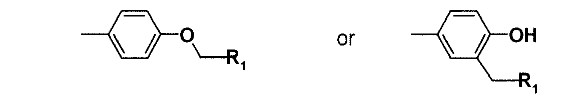

wherein Z₁ is O or S, and

X₂ is an α-amino acid having an aromatic residue on the C_{α} side chain, or an amino acid unit selected from Dab, Dpr, Dpm, His,(Bzl)HyPro, thienyl-Ala, cyclohexyl-Ala and t-butyl-Ala, the residue Lys of said sequence corresponding to the residue Lys⁹ of the native somatostatin-14.

By somatostatin analogue as used herein is meant a straight-chain or cyclic peptide derived from that of the naturally occurring somatostatin-14, comprising the sequence of formula I and wherein additionally one or more amino acid units have been omitted and/or replaced by one or more other amino acid radical(s) and/or wherein one or more functional groups have been replaced by one or more other functional groups and/or one or more groups have been replaced by one or several other isosteric groups. In general the term covers all modified derivatives of the native somatostatin-14 comprising the above sequence of formula I which have binding affinity in the nM range to at least one somatostatin receptor subtype as defined hereinafter.

Preferably, the somatostatin analogue is a compound in which the residues at positions 8 through 11 of the somatostatin-14 are represented by the sequence of formula I as defined above.

Particularly preferred are compounds of formula III 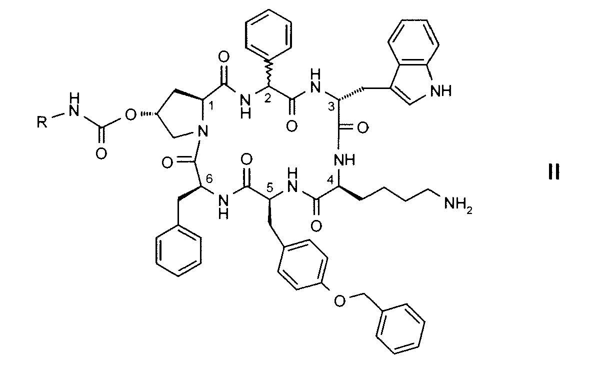 wherein the configuration at C-2 is (R) or (S) or a mixture thereof, and
wherein R is NR₁₀R₁₁C₂₋₆alkylene or guanidine-C₂₋₆alkylene, and each of R₁₀ and R₁₁ independently is H or C₁₋₄alkyl,
in free form, in salt form or protected form.

Preferably R is NR₁₀R₁₁C₂₋₆alkylene. Preferred compounds of formula II are the compounds wherein R is 2-amino-ethyl, namely cyclo[{4-(NH₂-C₂H₄-NH-CO-O-)Pro}-Phg-DTrp-Lys-Tyr(4-Bzl)-Phe] (referred herein to as Compound A) and cyclo[{4-(NH₂-C₂H₄-NH-COO-)Pro}-DPhg-DTrp-Lys-Tyr(4-Bzl)-Phe], in free form, salt form or protected form. Phg means -HN-CH(C₆H₅)-CO- and Bzl means benzyl.

A compound of the invention may exist e.g. in free or salt form. Salts include acid addition salts with e.g. inorganic acids, polymeric acids or organic acids, for example with hydrochloric acid, acetic acid, lactic acid, aspartic acid, benzoic acid, succinic acid or pamoic acid. Acid addition salts may exist as mono- or divalent salts, e.g. depending whether 1 or 2 acid equivalents are added. Preferred salts are the lactate, aspartate, benzoate, succinate and pamoate including mono- and di-salts, more preferably the aspartate di-salt and the pamoate monosalt.

The polymer of the composition of the invention may be a synthetic or a natural polymer. The polymer may be either a biodegradable or a combination of biodegradable and non-biodegradable polymers, preferably a biodegradable polymer may be used. By "polymer" is meant a homopolymer or a copolymer.

As used herein, "biodegradable" means a material that should degrade by bodily processes to products readily disposable by the body and should not accumulate in the body.

Suitable polymers include
(a) linear or branched polyesters which are linear chains radiating from a polyol moiety, e.g. glucose,
(b) polyesters such as D-, L- or racemic polylactic acid, polyglycolic acid, polyhydroxybutyric acid, polycaprolactone, polyalkylene oxalate, polyalkylene glycol esters of acids of the Kreb's cycle, e.g. citric acid cycle, and the like and combinations thereof,
(c) polymers of organic ethers, anhydrides, amides, and orthoesters,
(d) copolymers of organic esters, ethers, anhydrides, amides, and orthoesters by themselves or in combination with other monomers.

The polymers may be cross-linked or non-cross-linked. Usually not more than 5%, typically less than 1% are cross-linked.

The preferred polymers of this invention are linear polyesters, and branched chain polyesters. The linear polyesters may be prepared from the α-hydroxy carboxylic acids, e.g. lactic acid and glycolic acid, by condensation of the lactone dimers, see e.g. US 3,773,919, the contents of which are incorporated herein by reference. The preferred polyester chains in the linear or branched (star) polymers are copolymers of the α-carboxylic acid moieties, lactic acid and glycolic acid, or of the lactone dimers. The molar ratios of lactide: glycolide of polylactide-co-glycolides preferably used according to the invention is preferably from about 95:5 to 5:95, e.g. 75:25 to 25:75, e.g. 60:40 to 40:60, with from 55:45 to 45:55, e.g. 52:48 to 48:52, e.g. 50:50.

Linear polyesters, e.g. linear polylactide-co-glycolides (PLG), preferably used according to the invention have a weight average molecular weight (Mw) between about 1,000 and about 50,000 Da, e.g. about 10,000 Da, and a polydispersity M_{w}/Mₙ e.g. between 1.2 and 2. The intrinsic viscosities of linear polymers of Mw 1000 to 50,000 are 0.05 to 0.6 dl/g, 0.1% in chloroform (at 25°C) Suitable examples include e.g. those commonly known and commercially available as Resomers® from Boehringer Ingelheim, in particular Resomers® RG, e.g. Resomer® RG 502, 502H, 503, 503H.

Branched polyesters, e.g. branched polylactide-co-glycolides, preferably used according to the invention may be prepared using polyhydroxy compounds e.g. polyol e.g. glucose or mannitol as the initiator. These esters of a polyol are known and described e.g. in GB 2,145,422 B, the contents of which are incorporated herein by reference. The polyol contains at least 3 hydroxy groups and has a molecular weight of up to 20,000 Da, with at least 1, preferably at least 2, e.g. as a mean 3 of the hydroxy groups of the polyol being in the form of ester groups, which contain poly-lactide or co-poly-lactide chains. Typically 0.2% glucose is used to initiate polymerization. The branched polyesters (Glu-PLG) have a central glucose moiety having rays of linear polylactide chains, e.g. they have a star shaped structure.

The branched polyesters having a central glucose moiety having rays of linear polylactide-co-glycolide chains (Glu-PLG) may be prepared by reacting a polyol with a lactide and preferably also a glycolide at an elevated temperature in the presence of a catalyst, which makes a ring opening polymerization feasible.

The branched polyesters having a central glucose moiety having rays of linear polylactide-co-glycolide chains (Glu-PLG) preferably have a weight average molecular weight M_{w} in the range of from about 1,000 to 55,000, preferably 20,000, e.g. 10,000 Da, and a polydispersity e.g. of from 1.1 to 3.0, e.g. 2.0 to 2.5. The intrinsic viscosities of star polymers of M_{w} 10,000 to M_{w} 50,000 are 0.05 to 0.6 dl/g in chloroform. A star polymer having a M_{w} of 50,000 has a viscosity of 0.5 dl/g in chloroform.

The desired rate of degradation of polymers and the desired release profile for compounds of the invention may be varied depending on the kind of monomer, whether a homo- or a copolymer or whether a mixture of polymers is employed.

A mixture of polymers may comprise at least two different kinds of polymers, e.g. as listed under (a) to (e) above, or two polymers of the same polymer class with different properties. For example, a mixture of polymers may comprise a polymer having a medium weight average molecular weight, e.g. from about 30,000 to about 50,000 Da, e.g. of about 20,000 Da, and of a polymer having a low weight average molecular weight, e.g. of about 2.000 to about 20,000 Da, e.g. of about 10,000 Da.

Preferably, the polymer matrix comprises a linear and/or branched polylactide-co-glycolide. More preferably, the polymer matrix comprises a Resomer® RG and/or a star polylactide-co-glycolide polymer having a weight average molecular weight of about 10,000 Da and/or a star polylactide-co-glycolide polymer having a weight average molecular weight of about 50,000 Da. The ratio of linear to branched polylactide-co-glycolide preferably is 0 : 100 to 100 : 0, e.g. 50 : 50 to 25 : 75 to 75 :25.

In a particularly preferred embodiment the biodegradable polymer (e.g. PLA100 or PLGA50:50) has an inherent viscosity of 0.15-0.45dL/g (0.1% in CHCl3, 25°C).

In another aspect the invention provides a process for preparing a injectable in situ forming depot formulation comprising the steps:
(i) dissolving a biodegradable polymer, e.g. PLA or PLGA, in the solvent, e.g. an end-capped PEG with a solidification point between 8° to 20°C,
(ii) adding a pharmaceutically active agent and optionally an additive to achieve a solution or suspension,
   In case a suspension is obtained: continue with step iii)
(iii) DS particle size reduction by appropriate process, e.g. jet milling, ultrasound, high pressure homogenization, rotor- stator mixer (ultraturrax).

Alternatively, if the drug substance is soluble, steps (i) and (ii) can be switched.

The drug substance is dissolved or dispersed in the polymeric solution at a temperature where the solvent is liquid, conveniently e.g. room temperature. The dispersed drug substance may for instance be homogenized first by ultraturrax and then by ultrasound under cooling to expected particle size. A sterile product can be obtained by aseptic manufacturing or terminal sterilization.

This injectable in- situ depot shows an alternative controlled release formulation, easier in manufacturing and application than existing products. Compared to existing injectable in situ forming depots on polymer basis the depot formulation of the present invention is particularly useful as "ready to use devices" because with no re-suspending step is required prior to injection. The depot formulation of the present invention is the first formulation to be ready for injection after equilibration to room temperature. Dependant on viscosity the needle size can be adapted and drug load up to10%, up to 7-5% or up to 5% as suspension is possible.

The depot formulation of the present invention have advantageous properties: they have low hemolysis and toxicity potential and show good local tolerability at the injection site in rabbits. Sustained release systems in accordance with the present invention improve patience compliance and life standard. Furthermore the manufacturing process is simple and cheap and no organic solvent is required.

In one preferred embodiment of the present invention, no organic solvent is used during process for preparing the formulation of the present invention and hence no organic solvent is present in the formulation.

The depot formulation of the invention may be stored e.g. in prefilled syringe or other suitable containers, auto- injection device, vial and syringe over an extended period of time without sedimentation at a temperature below the melting point of the solvent such as e.g. the end-capped PEG.

The implant formed after injection into the body may release the active agent over an extended period of time. The desired release profile may depend on the kind of monomer, whether a homo- or a co-polymer or whether a mixture of polymers is employed. The release period may range from 1 up to 12 weeks, e.g. 1 to 8 weeks such as e.g. 4 weeks. Optionally an additive may be added to the polymer/solvent solution and/or to the polyethylene glycol/drug substance solution. The additive may improve the solubility of the polymer and the drug substance of the active ingredient. The co-solvent may further modulate the drug release in vitro or in vivo. The additive may be present in an amount of about 0.1 % to about 20% w/v, preferably from about 1% to about 5%. Examples of such additives include methanol, ethanol, propylene glycol , liquid surfactant such as poly(oxyethylene) sorbitan esters (Tweens) or glycerin polyoxyethylene ester of castor oil (Cremophor EL), lactic acid, acetic acid, glycerol, N,N dimethylacetamide, benzyl benzoate, polyoxyethylated fatty acid, lecithin, soybean oil , seaflower oil, vegetable oils, cotton sead oils, oligormers of poly(I-lactide) of poly(d,l lactide) of poly(lactide co-glycolide) or a mixture of these oligomers.

Details of suitable excipients for use in the compositions or process of the invention are described e.g. in the "Handbook of Pharmaceutical Excipients", Rowe, Sheskey and Weller, 4th Edition 2003.

In a further aspect of the invention the composition obtainable by the process of the present invention may be in liquid form at room temperature, e.g. a solution. After sterile filtration through a 0.22 micrometer filter the liquid composition, e.g. solution, may be placed in a syringe. Sterilization may also be achieved by another terminal sterilization with gamma irradiation at 20 to 30 kGy preferably at 25 kGy under cooled conditions, e.g. 2 to 8 °C or -70 °C. The sterilized solution may be injected through a needle, e.g. an up to 20 G needle, into the body subcutaneously or intramuscularly. Once in place the solvent, e.g. polyethylene glycol will dissipate and the polymer together with the pharmaceutically active agent solidifies to form the implant. Accordingly to the invention, preferably a prefilled syringe may be provided together with instructions for use.

The compositions of the invention are useful for treatment of the known indications of the particular active agent incorporated in the polymer in the indications as described on page 11 of WO02/010192. Preferably, the compositions of the invention are useful in the treatment of acromegaly and cancer, e.g. carcinoid tumor, Cushing's Disease.

The activity and the characteristics of the liquid compositions of the invention may be indicated in standard clinical or animal tests. Appropriate dosage of the composition of the invention will of course vary, e.g. depending on the condition to be treated (for example the disease type of the nature of resistance), the drug used, the effect desired and the mode of administration.

For compositions of the invention comprising a somatostatin analogue satisfactory results are obtained on administration, e.g. parenteral administration, at dosages on the order of from about 0.2 to about 60 mg, preferably from about 5 to about 40 mg per injection per month or about 0.03 to about 1.2 mg per kg animal body weight per month, administered once or in divided doses. Suitable monthly dosages for patients are thus in the order of about 0.3 mg to about 40 mg of a somatostatin analogue, e.g. Compound a pamoate. The composition may be administered every 2 to 3 months. Suitable dosages for every 3 months administration are about 1 mg to about 180 mg.

Following is a description by way of example only of processes and compositions of the invention.

**Table 1 Hemolysis in [%] of solvents (PEG500 DME, PEG 600 and NMP) tested in a female¹ donor and a male¹ donor**

| **Hemolysis [%] in female/ male donor** | **Dilutions with 0.9% NaCl solution** | | | | | | | |
|---|---|---|---|---|---|---|---|---|
| **solvent** | **0** | **1:2** | **1:4** | **1:8** | **1:16** | **1:32** | **1:64** | **1:128** |
| **PEG 600** | **13/ 8** | **1/ 0** | **0/ 0** | **1/ 0** | **1/ 0** | **1/ 0** | **1/ 0** | **1/ 0** |
| **NMP** | **54/ 28** | **10/ 0** | **1/ 0** | **1/ 0** | **1/ 0** | **1/ 0** | **1/ 0** | **1/ 0** |
| **PEG 500 DME** | **2/ 6** | **0/ 0** | **0/ 0** | **1/ 0** | **1/ 0** | **1/ 0** | **1/ 0** | **1/ 0** |

Table 1 summarizes values for the hemolytic activity of 3 solvents. PEG 500 shows lower hemolysis values of the pure solvent compared to PEG600 and NMP; NMP in a concentration of 1:2 still shows hemolytic activity compared to PEG 500 DME and PEG 600
Figure 1 demonstrates the *in-vitro* release of a 5% (drug substance base) loaded suspension with SOM230 over 48 days.
Figure 2 presents the *in-vitro* release profile of a 3.5% (drug substance base) loaded suspension with SOM230 over 48 days.

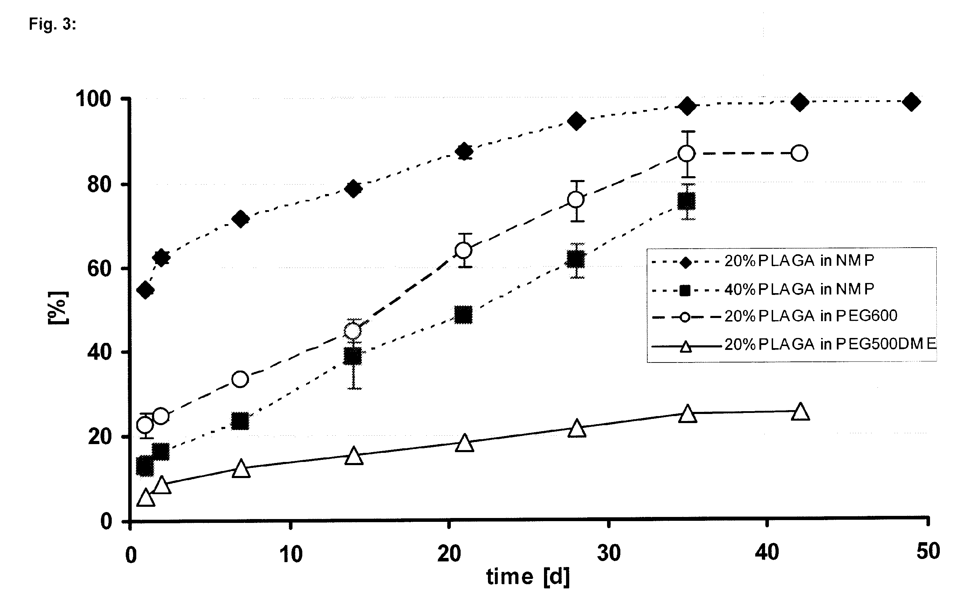

Figure 3 shows the release of methylene blue out of 4 formulations injected with a 1 ml syringe and a 23Gneedle, where a 20% PLGA50:50 solution in PEG500DME indicates a very low burst and constant sustained release over the observation time of 49days.

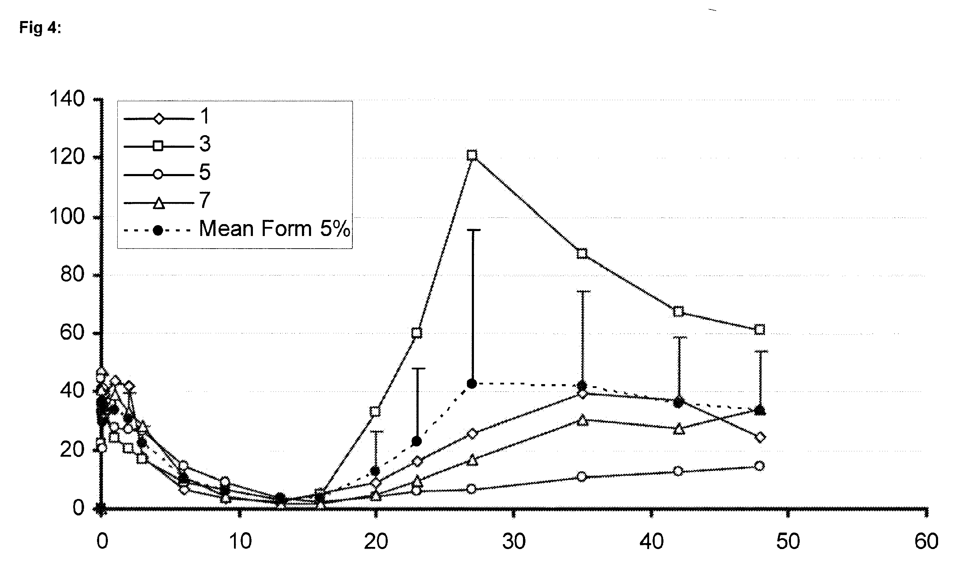

Figure 4 represents the *in-vivo* release of a 5% (drug substance base) loaded suspension with SOM230 over 48 days in rabbits.

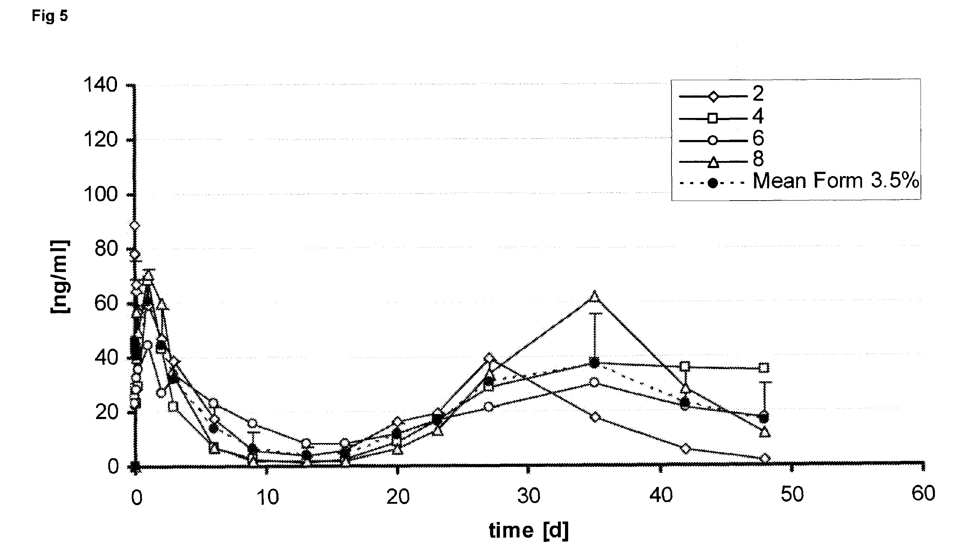

Figure 5 shows the *in-vivo* release profile in rabbits of a 3.5% (drug substance base) loaded suspension with SOM230 over 48 days.

### Example 1

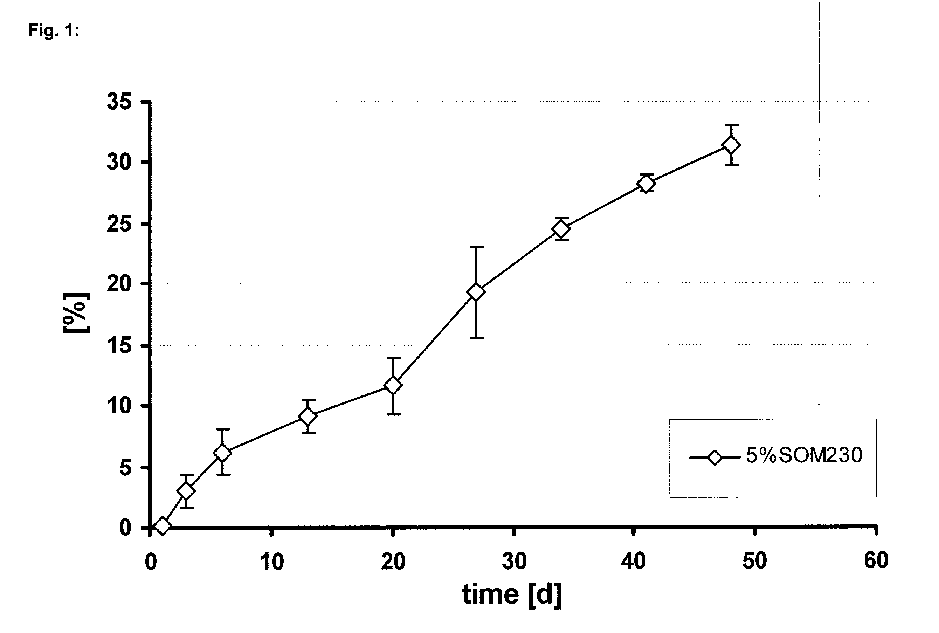

0.960g Poly (D,L-lactide-co-glycolide) 50:50 (20% w/w) is dissolved in 3.5062g Poly (ethylene glycol) dimethylether. 0.3404g gamma irradiated pharmaceutically active agent SOM230 pamoate (= 0.250 g free base = 5% w/w) is added to the polymer solution after sterile filtration (Millex GV, 0.22micrometer, Millipore, Zug, Switzerland) with 1 bar N2 pressure and dispersed in the solution by magnetic stirring. The dispersion is sonicated 2*5min with an ultrasound probe (hielscher UP400S, Ultrasound technology, Stuttgard, Germany) to a mean particle size of approx. 51 micrometer (determined with a Lasentec probe, Mettler-Toledo, Greifensee, Switzerland). 0.240 g formulation (+overfill = 0.333g) are filled in 1 ml syringes (BD, 1 ml syringe with Luer-Lok tip, Franklin Lakes, NJ, USA ) with a 22G needle (Sterican, 0.70x 0.30 BULB, B. Braun, Melsungen, Germany). The amount of injected formulation is adjusted to approx. 0.012g drug substance base. The in situ depot formulation is injected directly in 12mm cells (diameter) filled with 2ml PBS buffer pH 7.4 and placed in a USP 4 apparatus (Sotax, Allschwil, Switzerland) for *in-vitro* release. Buffer pH 7.4 is pumped through the apparatus with an Ismatec IP pump (Ismatec, Glattbrugg, Switzerland) at flow rate of 0.5ml/h. Samples are collected after 1, 3, 6, 13, 20, 27, 34, 41, 48 days and analyzed by HPLC. Figure 1 shows a sustained *in-vitro* release profile over 48 days of approx. 30% of the theoretical drug content with very low initial release (burst).

### Example 2

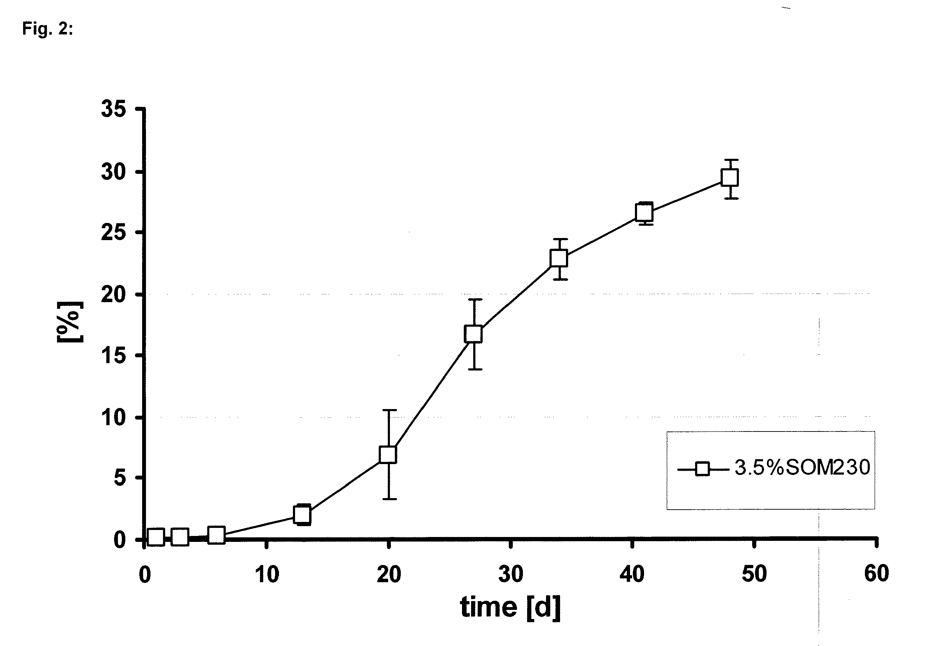

1.00139g Poly (D,L-lactide-co-glycolide) 50:50 (20% w/w) are dissolved in 3.75521g Poly (ethylene glycol) dimethylether. 0.2523g gamma irradiated pharmaceutically active agent SOM230 pamoate (= 0.250 g free base = 2.5% w/w) is added to the polymer solution after sterile filtration (Millex GV, 0.22micromreter, Millipore, Zug, Switzerland) with 1bar N2 pressure and dispersed in the solution by magnetic stirring. The dispersion is sonicated 2*5min with an ultrasound probe (hielscher UP400S, Ultrasound technology, Stuttgard, Germany) to a mean particle size of approx. 54micrometer (determined with a Lasentec probe,Mettler-Toledo, Greifensee, Switzerland). 0.3301 g formulation (+overfill = 0.420g) are filled in 1ml syringes (BD, 1ml syringe with Luer-Lok tip, Franklin Lakes, NJ, USA ) with a 22G needle (Sterican, 0.70x 0.30 BULB, B. Braun, Melsungen, Germany). The amount of injected formulation is adjusted to approx. 0.012g drug substance base. The in situ depot formulation is injected directly in 12mm cells (diameter) filled with 2ml PBS buffer pH 7.4 and placed in a USP 4 apparatus (Sotax, Allschwil, Switzerland) for *in-vitro* release. Buffer pH 7.4 is pumped through the apparatus with an Ismatec IP pump at flow rate of 0.5ml/h. Samples are collected after 1, 3, 6, 13, 20, 27, 34, 41, 48 days and analyzed by HPLC. Figure 2 shows a sustained *in-vitro* release profile over 48 days of approx. 30% of the theoretical drug content with very low initial release (burst).

### Example 3

0.048g pharmaceutically active agent (0.6% methylene blue) is added to 4 polymer solutions with different solvents. The first solution comprises 1.6002g Poly (D,L-lactide-co-glycolide) 50:50 (20% w/w) and 6.4345g Poly (ethylene glycol) dimethylether, the second solution includes 1.6004 Poly (D,L-lactide-co-glycolide) 50:50 (20% w/w) dissolved 6.4092g N-Methylpyrrolidine, the third solution of 3.2006g Poly (D,L-lactide-co-glycolide) 50:50 (40% w/w) in 4.818g N-Methylpyrrolidine and the last solution of 1.6012g Poly (D,L-lactide-co-glycolide) 50:50 (20% w/w) in Poly (ethylene glycol) 600.
0.400- 0.500 g of methylene blue loaded polymer solutions are injected (1ml syringe (BD 1ml with Luer-Lok tip, BD, Franklin Lakes, NJ, USA) and a 23G needle (BD Microlance 3,0.6x25mm, BD, S.A. Fraga, Spain) into 50ml polypropylene Falcon tubes (BD, Franklin Lakes, USA) with 25ml PBS buffer pH 7.4. The tubes are incubated in a water shaker bath (AD Krauth, Hamburg, Germany) at 37°C at very low frequency. Buffer replacement is performed at every sampling point. The samples are taken at t= 0, 1, 3, 7, 14, 21, 28, 35, 42 and 49 days and analyzed with a Varian Cary spectrophotometer (Darmstadt, Germany) at a wavelength of 665nm.

### Example 4

0.960g Poly (D,L-lactide-co-glycolide) 50:50 (20% w/w) is dissolved in 3.5062g Poly (ethylene glycol) dimethylether. 0.3404g gamma irradiated pharmaceutically active agent SOM230 pamoate (= 0.250 g free base = 5% w/w) is added to the polymer solution after sterile filtration (Millex GV, 0.22micrometer, Millipore, Zug, Switzerland) with 1bar N2 pressure and dispersed in the solution by magnetic stirring. The dispersion is sonicated 2*5min with an ultrasound probe (hielscher UP400S, Ultrasound technology, Stuttgard, Germany) to a mean particle size of approx. 51 micrometer (determined with a Lasentec probe, Mettler-Toledo, Greifensee, Switzerland). 0.240 g formulation (+overfill = 0.333g) are filled in 1ml syringes (BD, 1ml syringe with Luer-Lok tip, Franklin Lakes, NJ, USA ) with a 22G needle (Sterican, 0.70x 0.30 BL/LB, B. Braun, Melsungen, Germany). The amount of injected formulation is adjusted to approx. 0.012g drug substance base per animal. Four rabbits are tested on the *in-vivo* drug release of SOM230 pamoate. The in situ depot formulation is injected subcutaneously in the neck area of each rabbit. Blood samples (1-1.5 mL) were collected from *V. auricularis* into polypropylene syringes containing 1.6 mg potassium-EDTA (S-Monovette, Sarstedt AG, Sevelen, Switzerland). The samples were taken after 0 (=pre-dose), 30 min, 1, 2, 4, 6 hours, 1, 2, 3, 6, 9, 13, 16, 20, 23, 27, 35, 42 and 48 days. The plasma samples were analyzed for SOM230 using a competitive ELISA test.

### Example 5

1.00139g Poly (D,L-lactide-co-glycolide) 50:50 (20% w/w) are dissolved in 3.75521g Poly (ethylene glycol) dimethylether. 0.2523g gamma irradiated pharmaceutically active agent SOM230 pamoate (= 0.250 g free base = 2.5% w/w) is added to the polymer solution after sterile filtration (Millex GV, 0.22micromreter, Millipore, Zug, Switzerland) with 1bar N2 pressure and dispersed in the solution by magnetic stirring. The dispersion is sonicated 2*5min with an ultrasound probe (hielscher UP400S, Ultrasound technology, Stuttgard, Germany) to a mean particle size of approx. 54micrometer (determined with a Lasentec probe,Mettler-Toledo, Greifensee, Switzerland). 0.3301 g formulation (+ovefill = 0.420g) are filled in 1ml syringes (BD, 1ml syringe with Luer-Lok tip, Franklin Lakes, NJ, USA ) with a 22G needle (Sterican, 0.70x 0.30 BULB, B. Braun, Melsungen, Germany). The amount of injected formulation is adjusted to approx. 0.012g drug substance base per animal. Four rabbits are tested on the *in-vivo* drug release of SOM230 pamoate. The in situ depot formulation is injected subcutaneously in the neck area of each rabbit. Blood samples (1-1.5 mL) were collected from *V. auricularis* into polypropylene syringes containing 1.6 mg potassium-EDTA (S-Monovette, Sarstedt AG, Sevelen, Switzerland). The samples were taken after 0 (=pre-dose), 30 min, 1, 2, 4, 6 hours, 1, 2, 3, 6, 9, 13, 16, 20, 23, 27, 35, 42 and 48 days. The plasma samples were analyzed for SOM230 using a competitive ELISA test.

## Claims

1. An injectable in situ forming depot formulation comprising
i) a pharmaceutically active agent,
ii) a poly (ethylene) glycol with a molecular weight 450<Mw< 650Da and with chemically inert end groups which has a solidification point at a temperature between 8° to 20°C,
iii) a biodegradable polymer, and optionally
iv) an additive.

2. An injectable in situ forming depot formulation according to claim 1 wherein the inert end groups are alkoxy groups.

3. An injectable in situ forming depot formulation according to claim 1 or 2 wherein the polymer is PLA or a linear or branched PLGA.

4. An injectable in situ forming depot formulation according to claim 1 to 3 wherein the biodegradable polymer is Polylactide or a Poly (lactide-co-glycolide) with inherent viscosity of 0.15-0.60dL/g.

5. An injectable in situ forming depot formulation according to claim 1 to 4 wherein the formulation is free of organic solvents.

6. An injectable in situ forming depot formulation according to claim 1 to 5 wherein the pharmaceutically active agent is selected from small molecules, peptides, polypeptides, proteins, carbohydrates, oligonucleotides, RNA and DNA.

7. An injectable in situ forming depot formulation according to claim 1 to 6 wherein the pharmaceutically active agent is a bisphosphonate or a somatostatin analogue.

8. An injectable in situ forming depot formulation according to claim 7 wherein the pharmaceutically active agent is a cyclo[{4-(NH₂-C₂H₄-NH-CO-O-)Pro}-Phg-DTrp-Lys-Tyr(4-Bzl)-Phe] pamoate or di-aspartate.

9. A process for preparing a depot formulation comprising the steps:
i) dissolving a biodegradable polymer in an PEG with a molecular weight of at least 450 Da and less than 650 Da and with inert end groups which has a melting point <15°C and which is liquid at room temperature,
ii) adding a pharmaceutically active agent and optionally an additive to achieve a solution or suspension,
iii) and in case a suspension is obtained milling the formulation by an appropriate particle size reduction process to expected mean particle size.

10. A process for preparing a depot formulation comprising the steps:
i) dissolving a pharmaceutically active agent in an PEG with a molecular weight of at least 450 Da and less than 650 Da and with inert end groups which has a melting point <15°C and which is liquid at room temperature,
ii) adding a biodegradable polymer to achieve a solution or suspension,
iii) and in case a suspension is obtained milling the formulation by an appropriate particle size reduction process to expected mean particle size.

11. The process according to claim 9 or 10 wherein the process does not use an organic co-solvent.

12. The process according to claim 9 to 11 wherein the polymer is PLA or a linear or branched PLGA.

13. The process according to claim 9 to 12 wherein the biodegradable polymer has an inherent viscosity of 0.15-0.60dL/g.

14. The process according to claim 9 to 13 wherein the pharmaceutically active agent is selected from small molecules, peptides, polypeptides, proteins, carbohydrates, oligonucleotides, RNA and DNA.

15. The process according to claim 9 to 14 wherein the pharmaceutically active agent is a somatostatin analogue.

16. The process according to claim 9 to 15 wherein the pharmaceutically active agent is a cyclo[{4-(NH₂-C₂H₄-NH-CO-O-)Pro}-Phg-DTrp-Lys-Tyr(4-Bzl)-Phe] pamoate or di-aspartate.

17. A pharmaceutical composition comprising a pharmaceutically active agent wherein the composition is obtainable by the process according to claim 9 to 16.

18. A pharmaceutical composition according to claim 1 to 8 or 17 for injection.

19. The pharmaceutical composition according to any one of claims 1 to 8 or 17 wherein the active agent is released over 1 up to 12 weeks.

20. A prefilled syringe comprising the composition of any one of claims 1 to 8 or 17 and instructions to use.
